Europäisches Patentamt

European Patent Office

Office européen des brevets

(11) Publication number: **0 532 777 A1**

## EUROPEAN PATENT APPLICATION

(21) Application number: **91115800.4**

(22) Date of filing: **18.09.91**

(51) Int. Cl.5: **C12N 9/98**, C12N 9/36

(43) Date of publication of application:
**24.03.93 Bulletin 93/12**

(84) Designated Contracting States:
**AT BE CH DE DK ES FR GB GR IT LI LU NL SE**

(71) Applicant: **HAARMANN & REIMER CORP.**
**70 Diamond Road, P.O.Box 175**
**Springfield, New Jersey 07081(US)**

(72) Inventor: **Monticello, Daniel J.**
**721 Violet Street**
**Elkhart, Indiana 46514(US)**

(74) Representative: **Petrovicki, Wolfgang, Dr. et al**
**Bayer AG Konzernverwaltung RP Patente**
**Konzern**
**W-5090 Leverkusen 1 Bayerwerk (DE)**

(54) **Production of lysozyme containing particles.**

(57) Disclosed is a method for the preparation of lysozyme containing granules which involves applying the lysozyme to the surface of core particles in a fluidized bed reactor.

EP 0 532 777 A1

Rank Xerox (UK) Business Services
(3.10/3.5x/3.0.1)

## Background of the Invention

This invention relates to a procedure for making dry, dust free lysozyme containing particles and dry, dust-free particles containing a homogeneous mix of lysozyme together with other active and inactive components. Such particles are particularly useful as ingredients in processed or formulated foods; such as cheeses, communated meat products, salads and soups; components of antimicrobial dips, sprays or other treatments and various other food, cosmetic, pharmaceutical and industrial applications.

Lysozyme, now officially described as N-acetylhexosaminodase, has been assigned the designation 3.2.1.17 by the Commission on Enzymes. This enzyme, which has a molecular weight of approximately 14,300 to 14,600 and an isoelectric point at pH 10.7, can be obtained by crystallization from egg albumen.

Lysozyme is so named because of its ability to lyse or dissolve the cell walls of bacterial cells apparently by splitting or hydrolyzing the $\beta$(1-4) linkages between N-acetylmuramic acid and N-acetylglucosamine, which are parts of a long, complex sugar molecule found in the cell wall of many living bacterial cells. Because of the nature of the cell wall, Gram-positive bacteria are most susceptible to lysozyme. Because of its anti-bacterial activity, lysozyme has been described as being useful as a food preservative for controlling the growth of Clostridia in food products, cf. Carini et al, Microbiologie-Aliments-Nutrition, 1985, pp. 299-320.

While it is a very useful antimicrobial, the use of dry lysozyme has been somewhat limited by its propensity towards dusting and the difficulty with which it can be dissolved in water. These are serious drawbacks because of the allergenicity problems which can arise anytime a protein is inhaled into the lungs. Chronic exposure to such dust can lead to severe immune responses. In addition, currently available dry lysozyme is difficult to redissolve in water which prolongs the time required to work with this material.

Proctor and Cunningham report in CRC Critical Reviews in Food Science and Nutrition, Vol. 26, Issue 4 (1988), Pp. 359-395 that lysozyme is heat stable in acidic solutions and has been reported to withstand 100°C with little loss of activity. However, they also discuss a 25% inactivation of lysozyme at 70°C in borate buffer at pH 7.9 within 30 minutes. This survey article also describes the destruction of lysozyme in egg white at pH 8.0 in 15 minutes at 65°C whereas at pH 5 and 60°C, no loss of activity occurred in 60 minutes. It is also reported that lysozyme was found to be over 50 times more heat stable in phosphate buffer, pH 6.2, than in egg white at 62.5°C.

Lysozyme activity is also affected during its processing. Proctor et al report at page 365/366 that the type of container used to store lysozyme may affect its activity. Thus, in a study on the effect of Pyrex®, polypropylene and polyethylene containers it was found that lysozyme concentrations fell 40% after 24 hours in cold storage in Pyrex® and polypropylene containers. In another study, it was found that lysozyme adheres to glassware causing poor reproducibility in determination of its activity.

Good et al describe in U.S. Patent 4,689,297 the formulation of dust free enzyme particles by introducing a particulate core material into the production chamber of a fluidized bed reactor wherein the particles are sprayed with an aqueous slurry of an enzyme to provide, upon evaporation of the water, a dried layer of enzyme on the core particle. They describe an air inlet temperature of at least 50°C which works well with enzymes which are thermally stable but which provides no assurance that the heat and environmentally labile lysozyme could be handled in a similar manner.

## Summary of the Invention

The present invention involves the preparation of dust free lysozyme containing particles which method comprises spraying an aqueous slurry of lysozyme along with core particles into the reaction chamber of a fluidized bed reactor to thereby evaporate residual water and leave residual dried lysozyme coated on the particulate core material to thereby provide the lysozyme containing particles.

## Description of the Invention

The lysozyme containing particles are formed in the reaction chamber of a fluidized bed reactor. Typically, such devices comprise a dryer consisting of a circular product chamber that has a porous grid on the bottom and is open on the top so as to be put up against a conically shaped expansion chamber of a larger diameter than the circular product chamber. In operation, as the velocity of air passing up through the chamber is increased, a point is reached where particles resting on the porous grid are suspended in the airflow as a fluid, hence the terms "fluidization" and "fluid bed dryer". The particles are lifted by the upward force of the air out of the product chamber into the expansion chamber where the air expands and the upward force per unit of area is reduced. This allows the particles to fall back into the product chamber and start the cycle over.

The initial step in the present method involves introducing an aqueous slurry of lysozyme into the reaction chamber of the fluidized bed reactor and suspending it in the stream of air. Along with the lysozyme slurry there is injected a mass of core particles. The lysozyme is repeatedly contacted with a foreign material at elevated temperatures during formation of the particles in the fluidized bed reactor. Typically, the air temperature inside the reactor is from 40° to 60°C.

The core particles preferably consist of a highly hydratable material, i.e. a material which is readily dispersible or soluble in water. The core material should either disperse (fall apart by failure to maintain its integrity) or solubilize by going into a true solution. Clays (bentonite, kaolin), non-pareils and agglomerated potato starch are considered dispersible. Non-pareils are spherical particles consisting of a solid core that has been rounded into a spherical shape by binding layers of powder to the core in a rotating spherical container. Salt particles (NaCl crystals, NaCl rock salt, $NaHCO_3$) are considered soluble. Also suitable are agglomerated trisodium citrate, pan crystallized NaCl flakes, bentonite granules and prills, bentonite/kaolin/diatomaceous earth disk pelletized granules and sodium citrate crystals. The core particle is of a material which is not dissolved during the subsequent spraying process and is typically of a particle size of from 150 to 2,000 microns (100 mesh to 10 mesh on the U.S. Standard Sieve Series) in its longest dimension.

Typically, the lysozyme or blend of lysozyme and another active or inactive material is dispersed in water to a level of 15 to 25% solids (w/w). The dispersion, including any optional binders, salts or plasticizers must have a viscosity low enough (typically 10 to 5,000 cps at room temperature) to be pumped and atomized for effective spray coating. The lysozyme preparation is applied to the surface of the core by fluidizing the core particle in a flow of air whereupon a solution containing the lysozyme and optionally other solids is atomized and sprayed into the fluidized bed. The atomized droplets contact the surface of the core particles leaving a film of solids adhering to the surface of the particles when the water is evaporated.

There are numerous reports in the literature describing useful combinations of lysozyme and other active components. The present invention can be used to prepare dry, dust free, homogeneous mixtures of these components with lysozyme despite the known instability of this enzyme. Other active ingredients which can be added to the lysozyme slurry and be co-deposited on the core particle to enhance their antimicrobial activity include complexing agents such as citric acid and EDTA, proteinogenic amino acids such as cystine, alanine, tyrosine and glycine, certain lytic peptides such as a cecropin or sarcotoxin, and organic acids such as citric acid, malic acid and lactic acid.

Once prepared, the lysozyme containing particles can be used to disinfect and/or preserve a variety of foods. Lysozyme has been used to preserve bacon, fish cakes, sausages, fresh vegetables, fruit, tofu bean curd, milk and various other dairy products. Lysozyme is used commercially to prevent the spoilage of aged cheeses (late-blowing). In practice, the granular material can be readily dissolved in water, or added directly to the food products during blending, since it will dissolve and form a homogeneous suspension.

The method of practicing the present invention is further illustrated by the following examples:

## Example I

Laboratory scale experiments were performed using a Uni-Glatt laboratory model fluid bed dryer with variable air temperature and flow through the bed. The device has a 6 inch Wurster insert which consists of a container (5 1/2" diameter by 6 1/2" height) for the core material that fits against the bottom of the device's expansion chamber. The plate on the bottom of the Wurster has holes in it to distribute the air through the bed with the holes in the center being of a larger diameter than the rest of the holes in the plate. A cylindrical hollow tube (2 3/4 inches diameter by 6 inches length) called a partition is suspended above these larger diameter holes creating a higher airflow up through the partition than up around the outside of the partition. The air flow is adjusted based on the quantity and density of the core particles so that the particles flow up inside the partition into the expansion chamber then fall back down outside the partition into the area with less airflow while the bed is kept fluidizing and drying. This difference in airflow creates a circular upward and downward movement of the particles. The spray nozzle is installed at the bottom of the partition pointed upwards. This arrangement keeps the atomized liquid co-current with the motion of the cores being coated and results in a smooth coating. The speed of the circular flowing motion of the cores is adjustable by regulating the amount of air going through the partition and the amount of air going around the outside of the partition. The droplet size of the atomized enzyme solution spray is adjusted by adjusting the liquid pumping rate and the air pressure for atomization. The process can be accelerated by using counter current downward spray without using the Wurster column.

The height of the Wurster insert partition is adjusted vertically from 1/4 to 3/4 inch up from the bottom plate. When denser core materials are used, up to 3/4 of the holes outside the partition are blocked off to provide a higher linear velocity for the air to lift the particles up through the inside of the partition and maintain a smooth circulation of material through the spraying area. The total air flow is adjusted to get good flow of cores through the partition and keep the bed outside the partition fluidized. Inlet air temperature is adjusted up to a maximum of 75°C so that the outlet as well as particle temperatures are below 50°C. Based on the reported properties of lysozyme in neutral pH solutions, it was to be expected that these conditions would be too harsh for survival of the lysozyme activity. Unexpectedly, the lysozyme activity was essentially unchanged after granulation, as shown in Example II. Typical outlet temperatures during the coating process are 25° to 40°C. The solids level of lysozyme slurry sprayed was 15 to 25% of the total (w/w). Feed rate varied from 5 ml/min to 20 ml/min. Atomization air pressure ranged from 1.0 to 1.5. bar. A typical dry weight gain of the core particle after lysozyme coating is 10 to 20% (w/w) depending on the final activity desired.

EXAMPLE II

a. Dust-free antimicrobial product including lysozyme and/or chelating agents.

Four spray coating runs were made to prepare a dust-free granular product from spray dried lysozyme powder. Lysozyme was spray coated onto NaCl in the Uni-Glatt fluid bed dryer as described above using the raw materials set out in Table 1.

Product AMP 46 is a simple formulation of lysozyme with an additional protein source, Maltrin 100, which can be added to enhance the binding of the lysozyme to the core particles. Another simple formulation, AMP 44, contains lactose to dilute the lysozyme in order to provide a product having a reduced specific activity. Sample AMP 50 illustrates the incorporation of sodium citrate, a chelating and antimicrobial agent, into the lysozyme granule. The experiment designated as AMP 51 demonstrates the incorporation of EDTA into the lysozyme containing granules. Regardless of the additional component, essentially all of the lysozyme activity was recovered in spite of its art recognized instability.

Table 1

|  |  |  | Solids in Spray Liquid |  |  |  |  |
| Sample | NaCl | Lysozyme | Maltrin 100 | Lactose | Sodium Citrate | EDTA | % Theoretical Recovery |
|--------|------|----------|-------------|---------|----------------|------|------------------------|
| AMP 46 | 1000 | 100 | 11 | -- | -- | -- | 105 |
| AMP 49 | 1000 | 100 | 12.5 | 12.6 | -- | -- | 98 |
| AMP 50 | 1000 | 100 | 12.5 | 12.6 | 33.6 | -- | 93 |
| AMP 51 | 1000 | 100 | 12.5 | 12.6 | -- | 20 | 105 |

The resulting granules were fairly uniform in appearance. Remaining lysozyme activity was determined by dissolving the particles in phosphate buffer (50 mM, pH 6.2) and determining enzyme activity using a standard lysozyme assay. Table 1 shows that the granulated material retained the lysozyme activity during and after the granulization process. Table 2 indicates that the lysozyme in these granules was more stable than spray dried lysozyme stored under similar conditions.

Table 2

| Lysozyme Activity (U/mg) After 24 Weeks at Room Temperature | | | | |
|---|---|---|---|---|
| Sample | Initial Activity | Final Activity | % Residual | % of Control |
| Lysozyme Powder | 48 | 17 | 35 | --- |
| AMP-48 | 4.5 | 2.2 | 53 | 151 |
| AMP-49 | 4.2 | 2.6 | 62 | 177 |
| AMP-50 | 3.8 | 2.9 | 76 | 217 |
| AMP-51 | 4.4 | 2.7 | 61 | 174 |

These results show that the lysozyme activity was not destroyed in any of the granulation procedures, and that in this form lysozyme is significantly more stable than the traditional spray dried product.

**Claims**

1. A method for the preparation of lysozyme containing particles which comprises spraying an aqueous slurry of lysozyme along with core particles of a hydratable material into the reaction chamber of a fluidized bed reactor to thereby evaporate residual water and leave residual dried lysozyme coated on the particulate core material to thereby provide the lysozyme containing particles.

2. The method of Claim 1 wherein the air temperature inside the reaction chamber is from 40° to 60°C.

3. The method of Claim 1 wherein the core particles are comprised of a highly hydratable material.

4. The method of Claim 3 wherein the hydratable material is composed of a clay, a non-pareil, agglomerated potato starch, a salt particle, agglomerated trisodium citrate, a bentonite granule or prill, a diatomaceous earth granule or a sodium citrate crystal.

5. The method of Claim 1 wherein the core particle is from 150 to 2,000 microns in its longest dimension.

6. The method of Claim 1 wherein the aqueous slurry contains from 15 to 25 weight percent lysozyme.

7. The method of Claim 1 wherein sufficient lysozyme is deposited on the surface of the core particle to provide a dry weight gain of from 10 to 20%.

8. The method of claim 1 wherein the slurry contains inactive and/or antimicrobially active components other than lysozyme.

9. The method of claim 8 wherein there is included in the slurry an antimicrobially active component selected from the group of complexing agents, proteinogenic amino acids, bacteriocins, lytic peptides and organic acids.

10. A method for the preparation of spherical granules having lysozyme activity which method comprises spraying an aqueous slurry of lysozyme having a concentration of from 15 to 25 weight percent lysozyme into the reaction chamber of a fluidized bed reactor in which is suspended, at a temperature of from 40 to 60°C, core particles of a hydratable material, to thereby deposit a layer of dry lysozyme on the surface of the core particles.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 193 829 (MILES INC) * Whole document * | 1-10 | C12N9/98 C12N9/36 |
| D | & US-A-4689297 | | |
| Y | WORLD PATENTS INDEX Week 7321, Derwent Publications Ltd., London, GB; AN 73-30179U (21) & JP-B-48 016 188 (TAKEDA CHEMICAL INDS LTD) * abstract * | 1-10 | |
| A | WORLD PATENTS INDEX LATEST Week 8202, Derwent Publications Ltd., London, GB; AN 82-002885 (02) & RO-A-71 592 (BIOFARM INTR MED) 22 July 1980 * abstract * | 1,10 | |
| Y | WO-A-9 106 638 (GENENCOR INTERNATIONAL, INC.) * Whole document * | 1-10 | |
| Y | WORLD PATENTS INDEX LATEST Week 8946, Derwent Publications Ltd., London, GB; AN 89-337033 (46) & JP-A-1 252 285 (AGENCY OF IND SCI TECH ET AL.) 6 October 1989 * abstract * | 1-10 | TECHNICAL FIELDS SEARCHED (Int. Cl.5) C12N C12M |
| A | US-A-3 798 128 (SHINPEL MINATO ET AL.) * Whole document, in particular column 1 line 55 - 59 * | 1,10 | |
| Y | PATENT ABSTRACTS OF JAPAN vol. 10, no. 366 (C-390)(2423) 6 December 1986 & JP-A-61 162 185 ( NAGASE SEIKAGAKU KOGYO K. K. ) 22 July 1986 * abstract * | 1,10 | |
| | -/-- | | |

The present search report has been drawn up for all claims

| Place of search | Date of completion of the search | Examiner |
|---|---|---|
| BERLIN | 04 MAY 1992 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

EUROPEAN SEARCH REPORT

Application Number

EP   91 11 5800
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-2 002 780 (LORENZO FERRARI ET AL.) <br> * Whole document, in particular page 1 line 56 - page 2 line 3 * <br> --- | 1,10 | |
| A | US-A-3 242 056 (R. DUBOIS-PREVOST) <br> * Whole document, in particular claims 5 - 6 * <br><br> ----- | 1,10 | |
| | | | TECHNICAL FIELDS SEARCHED (Int. Cl.5) |

The present search report has been drawn up for all claims

| Place of search | Date of completion of the search | Examiner |
|---|---|---|
| BERLIN | 04 MAY 1992 | JULIA P. |

EPO FORM 1503 03.82 (P0401)